# EUROPÄISCHE PATENTSCHRIFT

(11) **EP 0 579 796 B1**
(45) Veröffentlichungstag und Bekanntmachung des Hinweises auf die Patenterteilung: **25.09.1996**
(21) Anmeldenummer: 93902019.4
(22) Anmeldetag: 01.02.1993
(51) Int. Cl.: G01N 3/08, G01N 33/36

(54) **VERFAHREN UND VORRICHTUNG ZUR BESTIMMUNG VON FESTIGKEITSEIGENSCHAFTEN DER KETTFÄDEN EINER WEBKETTE**
METHOD AND DEVICE FOR DETERMINING THE STRENGTH OF WARP YARNS
PROCEDE ET DISPOSITIF POUR DETERMINER LES CARACTERISTIQUES MECANIQUES DES FILS DE CHAINE D'UNE CHAINE DE TISSAGE

(30) Priorität: 07.02.1992 CH 363/92
(43) Veröffentlichungstag der Anmeldung: 26.01.1994
(73) Patentinhaber: Stäubli AG, CH-8810 Horgen (CH)
(72) Erfinder: PLASCHY, Martin, CH-8634 Hombrechtikon (CH); KREBS, Walter, CH-7310 Bad Ragaz (CH)
(74) Vertreter: Ellenberger, Maurice
(86) Internationale Anmeldenummer: CH9300024
(87) Internationale Veröffentlichungsnummer: WO9316367

(56) Entgegenhaltungen:
- EP-A- 0 240 074
- EP-A- 0 403 988
- US-A- 4 805 276

## Beschreibung

Die Zugprüfung an textilen und technischen Garnen war schon immer ein wichtiger Test in der Qualitätskontrolle, und zwar sowohl bei Stapelfaser- als auch bei Filamentgarnen. Denn die Ergebnisse der Zugprüfung ermöglichen Rückschlüsse auf die Produktion und damit die Aufdeckung von Produktionsmängeln; Zugkraft- und Dehnungswerte geben Hinweise auf die Eignung des verwendeten Rohmaterials und erlauben Prognosen für die weitere Verarbeitung des Garns und für das Endprodukt.

Derartige Zugprüfungen werden heute off-line, im Textillabor, durchgeführt, indem die verschiedenen Garnpartien stichprobenartig untersucht werden. Ein bekanntes Gerät für die Zugprüfung an Garnen und Zwirnen ist die in der US-A-4,338,824 beschriebene Zugprüfanlage USTER TENSORAPID (USTER - eingetragenes Warenzeichen der Zellweger Uster AG), ein anderes Zugprüfgerät ist aus der EP-A-0 403 988 bekannt.

Mit modernen Prüfgeräten ausgerüstete Textillabors sind in Spinnereien sehr häufig und in Webereien eher selten anzutreffen. Der Grund für die geringe Verbreitung in Webereien ist darin zu suchen, dass die bekannten Prüfgeräte keine Prüfung an Kettbäumen erlauben, und dass die Festigkeitsdaten ungeschlichteter Garne nicht genügend Hinweise für das Laufverhalten an einer bestimmten Webmaschine mit einem bestimmten Artikel liefern. Dass aber auch in Webereien ein Interesse an Festigkeitsdaten besteht, wird durch die EP-A-0 240 074 bewiesen, die eine Vorrichtung zur Bestimmung der Festigkeitseigenschaften von Schussgarn betrifft, mit der in der Regel immer eine ganze Garnspule geprüft und dabei auch verbraucht wird.

Hingegen sind keine in den Gewebeherstellungsprozess integrierbare Zugprüfgeräte für Kettfäden bekannt, obwohl jeder Kettfadenbruch zu einem Maschinenstillstand führt, so dass jede Weberei ein vitales Interesse an der Qualitätskontrolle von Kettgarnen haben müsste.

Die vorliegende Erfindung, die ein Verfahren und eine Vorrichtung zur Bestimmung von Festigkeitseigenschaften der Kettfäden einer Webkette betrifft, soll eine möglichst rationelle, in den Gewebeherstellungsprozess integrierbare Bestimmung von Festigkeitseigenschaften ermöglichen. Dabei bedeutet "in den Gewebeherstellungsprozess integrierbar", dass die Bestimmung der Festigkeitseigenschaften nicht off-line in einem Textillabor, sondern on-line, möglichst nahe an einer Maschine des Gewebeherstellungsprozesses erfolgt. Dass diese Festigkeitsprüfung auch mit einem Minimum an dafür verbrauchtem Kettfadenmaterial verbunden sein soll, ist selbstverständlich.

Das erfindungsgemässe Verfahren ist dadurch gekennzeichnet, dass die Kettfäden als Fadenschicht aufgespannt, von der Fadenschicht einzeln abgeteilt und unter gleichzeitiger Messung von Zugkraft und/oder Dehnung gedehnt werden, und dass diese Operationen vor dem Webprozess in einer Stufe des Webereivorbereitungsprozesses erfolgen, in der die Kettfäden in Form einer vorbereiteten Kettfadenschicht vorliegen.

Die erfindungsgemässe Vorrichtung zur Durchführung dieses Verfahrens ist gekennzeichnet durch Mittel zum Aufspannen der Kettfadenschicht, Mittel zum Abteilen der Kettfäden und Mittel zur Messung von Zugkraft und/oder Dehnung der abgeteilten Kettfäden, wobei die genannten Mittel zum Aufspannen der Kettfadenschicht Bestandteil einer Webkettenvorbereitungsmaschine, vorzugsweise einer Knüpfanlage oder einer Einziehanlage bilden.

Die Erfindung geht von der neuen Erkenntnis aus, dass für eine rationelle Festigkeitsprüfung die Kettfäden als Fadenschicht vorbereitet werden müssen, von welcher sie einfach separiert und der Messvorrichtung vorgelegt werden können. Zur Realisierung dieser Erkenntnis macht sich die Erfindung die Tatsache zunutze, dass eine derartige vorbereitete Fadenschicht im Webereivorbereitungsprozess mehrmals vorhanden ist. So beispielsweise beim Knüpfen und beim Einziehen, wo die Kettfäden jeweils auf einem sogenannten Knüpfgestell oder auf einem Einziehrahmen aufgespannt sind. Da sowohl beim Knüpfen als auch beim Einziehen immer ein Abschneiden der Kettfäden erfolgt, stellen diese Operationen eine ideale Schnittstelle für die Festigkeitsprüfung dar.

Bekanntlich werden die Kettfäden beim Knüpfen und beim Einziehen vom jeweiligen Gestell oder Rahmen abgearbeitet und dabei geknotet beziehungsweise eingezogen, wobei an einem der Klemmelemente der Aufspannvorrichtung für die weitere Verarbeitung nicht mehr benötigte Fadenreste verbleiben. An diesen Fadenresten kann nun die Festigkeitsprüfung vorgenommen werden, wodurch auch die Forderung nach minimalem Materialverbrauch optimal erfüllt ist.

Das erfindungsgemässe Verfahren eröffnet neben der Qualitätskontrolle von Kettgarnen noch folgende Möglichkeiten: Indirekte Qualitätskontrolle des Schlichtprozesses, Vergleich der auslaufenden und der neuen Webkette beim Knüpfprozess, Ermittlung des Zusammenhangs zwischen den Festigkeitswerten und der Anzahl von Kettfadenbrüchen, und optimale Einstellung der Webmaschine anhand der ermittelten Festigkeitswerte. Weil die Festigkeitswerte zwischen geschlichteten und ungeschlichteten Garnen erfahrungsgemäss stark voneinander abweichen, ist die Anwendung des erfindungsgemässen Verfahrens auch dann sinnvoll, wenn am ungeschlichteten Garn eine Festigkeitsprüfung vorgenommen wurde.

Erfahrungen aus der Praxis beim Knüpfprozess haben gezeigt, dass teilweise die Kettfäden in der Mitte der Webkette wesentlich stärker verschlichtet sind als am Rand. Dies deutet auf eine fehlerhafte Einstellung oder auf einen anderen Mangel der Schlichtmaschine hin. Derartige Mängel können durch Anwendung des erfindungsgemässen Verfahrens relativ frühzeitig und objektiv erfasst werden.

Selbstverständlich werden die mit dem erfindungsgemässen Verfahren ermittelten Festigkeitswerte in der Regel nicht mit den mit klassischen Zugprüfgeräten wie mit dem schon erwähnten USTER TENSORAPID ermittelten Normwerten vergleichbar sein. Das ist aber insoweit nicht von Bedeutung, als diese Normwerte an ungeschlichteten Garnen gewonnen werden und damit für den Verarbeiter geschlichteter Garne ohnehin nur beschränkt aussagefähig sind. Wesentlich ist, dass dieser Verarbeiter, also der Weber, ein Hilfsmittel erhält, das ihm für seine Verarbeitungsstufen, also für die Webereivorbereitung und für die Weberei, miteinander vergleichbare Festigkeitswerte liefert.

Nachfolgend wird die Erfindung anhand von Ausführungsbeispielen und der Zeichnungen näher erläutert; es zeigt:
- Fig. 1: eine schematische Draufsicht auf ein Knüpfgestell mit zwei auf diesem aufgespannten Webketten und mit einer mit einem erfindungsgemässen Festigkeitsprüfer ausgerüsteten Knüpfmaschine,
- Fig. 2: ein Blockschaltbild der Signalauswertungsschaltung; und
- Fig. 3: eine Perspektivdarstellung eines Ausschnitts eines Ausführungsbeispiels einer mit einem Dehnungsprüfer ausgerüsteten Knüpfmaschine.

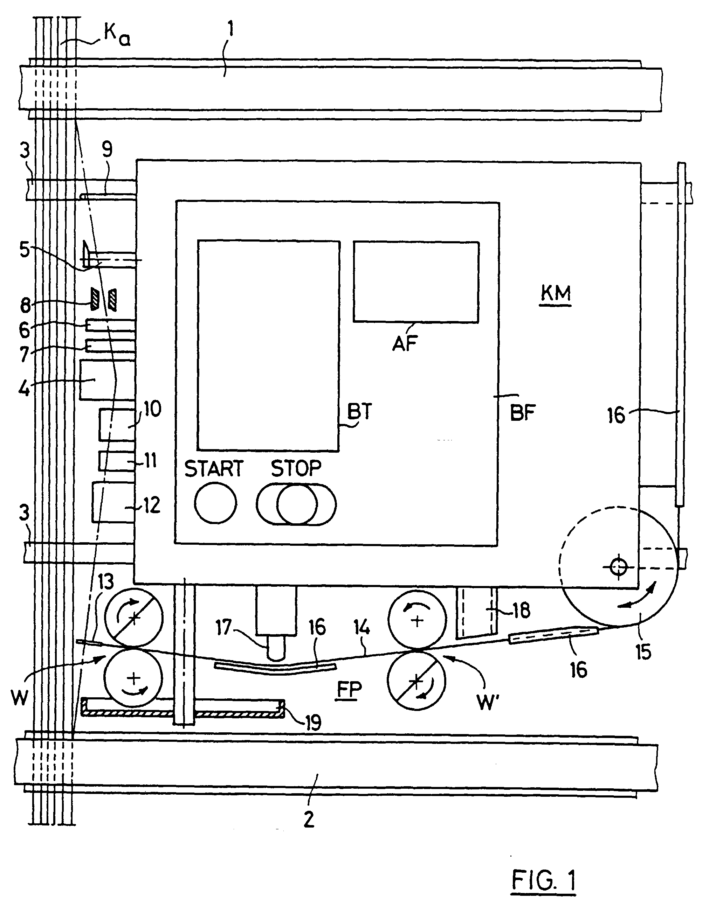

Fig. 1 zeigt eine schematische Draufsicht auf ein durch zwei Klemmschienen 1 und 2 symbolisiertes Knüpfgestell von der Art, wie es beispielsweise zusammen mit der Webkettenknüpfmaschine USTER TOPMATIC verwendet wird. Zwischen den beiden Klemmschienen 1 und 2 sind die Kettfäden Ka eines ablaufenden Kettbaumes aufgespannt, die mit einer entlang den Klemmschienen verfahrbaren Knüpfmaschine KM an die Kettfäden eines neuen Kettbaumes angeknüpft werden sollen. Die letzteren sind in einer unterhalb der Ebene der Kettfäden Ka liegenden Ebene zwischen einem zweiten Paar von Klemmschienen aufgespannt, so dass die Kettfäden Ka des ablaufenden Kettbaumes einerseits und die Kettfäden des neuen Kettbaumes andererseits je eine geordnet aufgespannte flächige Fadenschicht bilden. Diese Aufspannung ist beispielsweise in der US-A-4,805,276 beschrieben.

Die einzelnen Klemmschienen sind auf geeigneten Trägerarmen (nicht dargestellt) montiert, welche ihrerseits über Tragsäulen mit einem fahrbaren Grundgestell verbunden sind, so dass das Knüpfgestell in bekannter Weise einen fahrbaren Rahmen mit einem wannenartigen Oberteil bildet. Der letztere enthält neben den Klemmschienen zwei Führungsschienen 3 mit Zahnstangen, die zur Aufnahme bzw. Vorwärtsbewegung der Knüpfmaschine KM und zu Ausgleichsbewegungen der unteren Fadenschicht vorgesehen sind. Für die nachfolgende Beschreibung wird die Knüpfmaschine USTER TOPMATIC als bekannt vorausgesetzt; es wird in diesem Zusammenhang auf die schon erwähnte US-A-4,805,276 verwiesen, in welcher wesentliche Teile dieser Knüpfmaschine beschrieben sind. Im praktischen Einsatz wird die Knüpfmaschine KM an einer der beiden Seitenkanten der beiden Fadenschichten auf die Führungsschienen 3 aufgesetzt und anschliessend quer zu den Fadenschichten bewegt, wobei immer ein Faden Ka der ablaufenden Webkette mit einem Faden der neuen Webkette verknüpft wird.

Die Knüpfmaschine weist an ihrer der Bedienungsperson zugewandten oberen Deckfläche ein Bedienungsfeld BF auf, welches unter anderem eine Bedienungstastatur BT für die Eingabe verschiedener Funktionen und die Abfrage bestimmter Daten, eine Start- und eine Stoptaste, Anzeigeorgane für bestimmte Zustände sowie ein Anzeigefeld AF enthält.

Wenn eine Knüpfmaschine mit einem Festigkeitsprüfer ausgerüstet werden soll, dann ist eine der schwierigsten zu lösenden Aufgaben das Platzproblem, da der Abstand zwischen den Klemmschienen 1 und 2 durch die Platzverhältnisse im Websaal vorgegeben ist und praktisch nicht vergrössert werden kann.

Bei dem in Fig. 1 dargestellten Ausführungsbeispiel ist das Platzproblem so gelöst, dass die für die Knüpfoperation erforderlichen Elemente in der bei Knüpfmaschinen bekannten Art an der den Fadenschichten zugewandten Stirnseite der Knüpfmaschine KM angeordnet sind, wogegen der Festigkeitsprüfer FP an einer der beiden Längsseiten montiert ist, und zwar an derjenigen Längsseite, an der sich die nach dem Knüpfvorgang verbleibenden Fadenreste befinden. Eine andere Möglichkeit zur Lösung des Platzproblems bestünde darin, den Festigkeitsprüfer unterhalb der Knüpfelemente anzuordnen und zwar vorzugsweise in vertikaler Richtung.

Wie in der Beschreibungseinleitung erwähnt ist, müssen die Mittel zum Aufspannen der Kettfadenschicht nicht durch das Knüpfgestell für eine Webkettenknüpfmaschine gebildet sein, sondern bilden Bestandteil oder Zubehör einer Webkettenvorbereitungsmaschine, insbesondere auch einer Einziehmaschine. Da bei Einziehmaschinen zum Aufspannen der Kettfadenschicht Einziehrahmen verwendet werden, bei denen der Abstand zwischen den Klemmschienen ein Mehrfaches des Abstandes bei einem Knüpfgestell beträgt, ist klar, dass hier die Platzprobleme weniger kritisch sind. Es wird daher für den Fachmann einfach sein, den nachfolgend im Zusammenhang mit einer Knüpfmaschine beschriebenen Festigkeitsprüfer an einer Einziehmaschine anzuordnen. Dies gilt umso mehr, als der bei Knüpfmaschinen für das Abteilen der Randkettfäden verwendete Mechanismus auch bei Einziehmaschinen verwendet werden kann, was beispielsweise durch die WO-A-91/05099 belegt ist.

In Fig. 1 sind die folgenden von der USTER TOPMATIC her bekannten Knüpfelemente schematisch dargestellt, wobei die einzelnen Elemente in der Regel paarweise, je eines pro Fadenschicht, angeordnet sind: Eine Fadentrenngruppe 4, die im wesentlichen eine Abteilnadel für jede Fadenschicht, einen Zubringer zum Transport der Fäden zur Maschine und gegebenenfalls Sensoren für die Detektion von Doppelfäden (siehe US Patent 4,805,276) aufweist; ein eine Einziehnadel und einen Knüpferdorn aufweisendes gemeinsames Organ 5 für beide Fadenschichten; ein Paar von zwangsgesteuerten Fadenklemmen 6; zwei Scheren 7; einen sogenannten Knopfdreher 8; einen Knotenauszieher 9; eine Fadenkreuzsteuerung 10 und je einen Vorschubtaster 11 für die obere und die untere Fadenschicht.

Durch die Abteilnadel der Fadentrenngruppe 4 wird der jeweilige Randkettfaden abgeteilt und mit dem Zubringer in die in der Figur strichpunktiert eingezeichnete Position gebracht. Dann werden die Fäden von den Fadenklemmen 6 geklemmt und von den Scheren 7 geschnitten. Anschliessend werden sie vom Knopfdreher 8 übernommen, die Fadenklemmen 6 werden gelöst, die Fäden werden um den Knüpferdorn gewickelt und mit der Einziehnadel in den Knüpferdorn eingezogen. Nach der Bildung des Knotens wird mit dem Knotenauszieher 9 die lose Schlinge angezogen und die geknoteten Fäden werden abtransportiert.

Die Fadenkreuzsteuerung 10 tritt in den Fällen in Funktion, in denen ein Fadenkreuz benötigt wird. Ein derartiges Fadenkreuz, in der Regel ist es ein regelmässiges Fadenkreuz 1:1, ist dann erforderlich, wenn die Kettfadendichte eine gewisse Grenze überschreitet, oder wenn gewisse Spezialgarne, wie Flammen- oder Bouclegarne verarbeitet werden. Neben Fadenkreuzen 1:1 können aber auch unregelmässige Fadenkreuzsteuerungen abgearbeitet werden.

Der Vorschubtaster 11 ist ein mechanischer Taster für die obere und die untere Fadenschicht, welcher den Vorschub der Knüpfmaschine KM oder eine Bewegung der unteren Fadenschicht zur Knüpfmaschine hin auslöst. An der in Fig. 1 unteren Seite des Vorschubtasters 11 ist ein Organ eingezeichnet, welches nicht mehr für die eigentliche Knüpfoperationen erforderlich ist, sondern die Schnittstelle zum Festigkeitsprüfer bildet. Dieses Organ ist ein Fadenauslenker 12 pro Fadenschicht, welcher den zwischen der Fadenklemme 6 und der Klemmschiene 2 gespannten abgeteilten Randkettfaden aus seiner Fadenschicht in vertikaler Richtung auslenkt, und zwar aus der oberen Fadenschicht nach unten und aus der unteren Fadenschicht nach oben und dadurch den Abstand der Randkettfäden verkleinert. Diese Auslenkung der Randkettfäden erfolgt dazu, um sie einer im wesentlichen quer zu den Kettfäden bewegbaren Greifernadel 13 vorzulegen, welche die Kettfäden nach Beendigung der Knüpfoperation in den Festigkeitsprüfer FP einzieht.

Die Greifernadel 13 ist in der Art eines von Webmaschinen her bekannten Schlaufengreifers ausgebildet und weist an ihrem kopfseitigen Ende zwei Haken auf, von der der eine zum Einzug des oberen und der andere zum Einzug des unteren Randkettfadens bestimmt ist. Die Greifernadel 13 ist von einem flexiblen Transportband 14 getragen, welches von einem oszillierend bewegbaren Antriebsrad 15 angetrieben ist. Das Antriebsrad 15 ist an seinem Umfang mit stollenartigen Vorsprüngen versehen, welche in entsprechende Ausnehmungen des Transportbandes 14 eingreifen. Ein Antriebsmechanismus dieser Art ist von Greiferwebmaschinen her und aus der WO-A-91/10003 bekannt. Mit dem Bezugszeichen 16 sind mehrere entlang der Bewegungsbahn des Transportbandes 14 angeordnete Führungsmittel für dieses bezeichnet.

Der Festigkeitsprüfer FP ist von der in der EP-A-403 988 beschriebenen Art und enthält zwei voneinander beabstandete Walzenpaare W und W' zur Dehnung des von der Greifernadel 13 eingezogenen Fadenstücks, einen zwischen den Walzenpaaren W und W' angeordneten Kraftsensor 17, welcher vorzugsweise durch einen Piezoesensor gebildet ist, sowie eine in Einzugsrichtung der Fäden nach dem hinteren Walzenpaar W' angeordnete Saugdüse 18. Diese dient einerseits zur Uebernahme des Fadens von der Greifernadel 13 und zu dessen Spannen und andererseits zum Absaugen der bei der Festigkeitsprüfung abgerissenen Fadenenden. Alle diese Teile des Festigkeitsprüfers FP sind paarweise vorgesehen, und zwar für den Randkettfaden der oberen und für den Randkettfaden der unteren Kettfadenschicht. Im Bereich des vorderen Walzenpaares W ist eine beiden Teilen des Festigkeitsprüfers FP gemeinsame Ausziehscheibe 19 für die bei der Festigkeitsprüfung nicht abgerissenen Fadenteile vorgesehen.

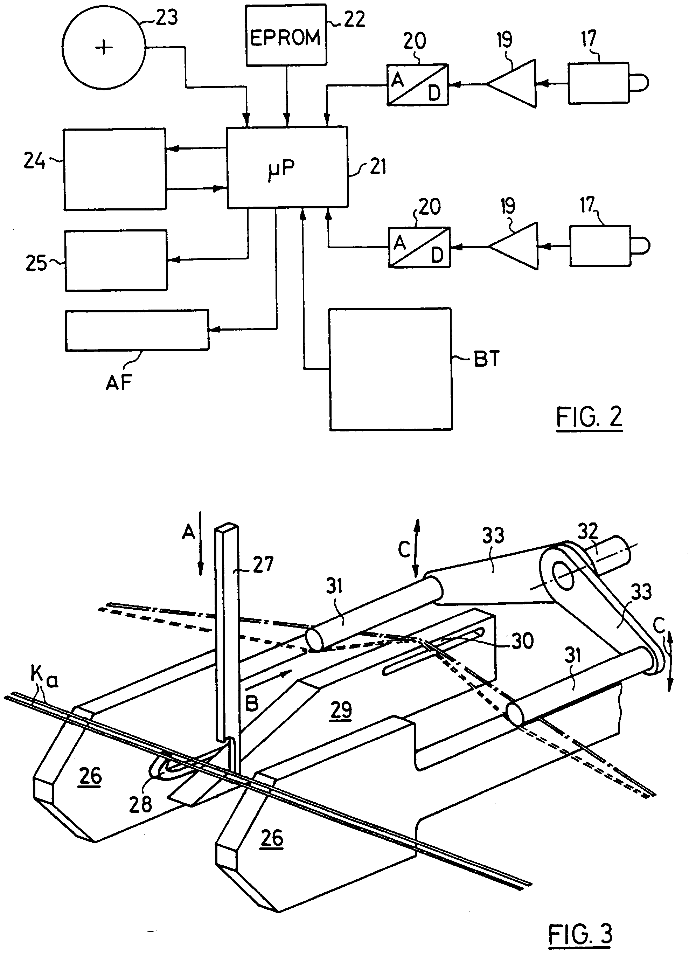

Jedes Walzenpaar W, W' besteht in bekannter Weise aus einer Anpresswalze und aus einer Transportwalze, von denen die letztere in ihrem den Faden klemmenden Bereich durch ein Kreissegment von der Form eines Halbkreises gebildet ist. Somit besteht zwischen den Walzen jedes Walzenpaares ein sich periodisch öffnender und schliessender Klemmspalt, der im geöffneten Zustand den Durchtritt von Greifernadel 13 und Transportband 14 und das Einziehen des Fadens ermöglicht, und im geschlossenen Zustand den Faden klemmt. Der Antrieb der Walzenpaare W, W' ist einerseits mit dem Antrieb des Antriebsrades 15 und andererseits mit dem Antrieb der Knüpfmaschine KM synchronisiert. Da der Antrieb der Knüpfmaschine KM variabel ist, ist die Drehzahl der Walzenpaare W, W' in der Regel nicht konstant, sondern ebenfalls variabel. Um bei verschiedenen Drehzahlen der Walzenpaare W, W' gemessene Festigkeitswerte miteinander vergleichen zu können, werden diese entsprechend umgerechnet. Diese Rechenoperation erfolgt in einem Mikroprozessor 21 (Fig. 2). Eine der Walzen des vorderen oder des hinteren Walzenpaares W bzw. W' oder das Antriebsrad 15 ist mit einem Inkrementalgeber 23 (Fig. 2) versehen.

Die kombinierte Operation Knüpfen und Festigkeitsprüfung erfolgt im wesentlichen in den folgenden fünf Phasen:
- Die erste Phase ist eine konventionelle Knüpfoperation mit den Stufen Abteilen, Transport der abgeteilten Fäden zur Knüpfmaschine, Klemmen, Schneiden, Knotenbildung. Die miteinander verknoteten Fadenenden werden vom Knotenauszieher 9 von den beiden Fadenschichten weggezogen. Bezogen auf Fig. 1 befindet sich dann oberhalb der Schere 7 der Knoten und unterhalb von dieser erstrecken sich relativ lange Fadenenden bis zur Klemmschiene 2. Gleichzeitig beginnt die Festigkeitsprüfung, die folgendermassen abläuft:
- Der Fadenauslenker 12 lenkt den betreffenden Randkettfaden aus seiner Fadenebene aus und bietet ihn der Greifernadel 13 an, welche durch das Transportband 14 von ihrer hinteren Stellung im Bereich der Absaugdüse 18 nach vorne gegen die Randkettfäden bewegt wird.
- Die Greifernadel 13 übernimmt kurz nach der Schneidoperation die angebotenen Fäden und zieht sie in den jeweiligen Teil des Festigkeitsprüfers FP ein, wobei zwischen den Walzen der Walzenpaare W, W' in dieser Phase ein offener Klemmspalt gebildet ist. In der hinteren Stellung der Greifernadel 13 wird der Faden der Absaugdüse 18 vorgelegt und von dieser übernommen und dadurch gespannt.
- Unmittelbar nach dem Aufbringen dieser Vorspannung wird der Faden zwischen den rotierenden Walzenpaaren W, W' geklemmt und anschliessend bis zur Reissgrenze gedehnt. Die dabei auftretende Kraft wird durch den Kraftsensor 17 gemessen.
- Nach dem Reissen des Fadens wird der abgerissene Fadenrest von der Absaugdüse 18 abgesaugt und damit aus dem Festigkeitsprüfer FP entfernt. Der vom vorderen Walzenpaar W geklemmte Fadenrest wird von der rotierenden Ausziehscheibe 19 erfasst und aus dem Walzenpaar herausgezogen. Er hängt dann von der Klemmschiene 2 frei nach unten, wie das auch bei den heute verwendeten Knüpfmaschinen ohne Festigkeitsprüfer der Fall ist. Damit ist der Festigkeitsprüfer FP für die Festigkeitsprüfung des nächsten Fadenpaares bereit, welches in der Zwischenzeit von der Knüpfmaschine abgearbeitet worden ist. Anstatt der Ausziehscheibe 19 kann zur Entfernung des im Festigkeitsprüfer FP verbliebenen Fadenrest auch eine Saugdüse vorgesehen sein.

Gemäss Fig. 2 sind die Ausgangssignale der beiden Piezosensoren 17 über einen Verstärker 19 und einen A/D-Wandler 20 an zwei Eingänge eines Mikroprozessors 21 geführt, dessen weitere Eingänge an einen beispielsweise durch ein EPROM gebildeten Programmspeicher 22, an den schon erwähnten Inkrementalgeber 23, an einen Speicher 24 und an die Bedienungstastatur BT angeschlossen, und dessen Ausgänge mit dem genannten Speicher 24, mit einem Datenausgang 25 und mit dem Anzeigefeld AF verbunden sind. Ausserdem sind Filter zur Filterung der Signale der Piezosensoren 17 vorgesehen.

Der Mikroprozessor 21 ermittelt aus den Signalen der Piezosensoren 17 für jeden Faden die Reisskraft, Werte für beispielsweise 2 Punkte der Kraft-Dehnungslinie, für deren Lokalisierung der Inkrementalgeber 23 erforderlich ist, sowie abgeleitete Grössen wie zum Beispiel die mittlere Reisskraft oder die Schwankungen der Reisskraft um einen Mittelwert. Alle diese Werte und Grössen werden im Speicher 24 abgelegt und können diesem entnommen und in beliebiger Zuordnung zur Anzeige gebracht werden. Letzteres ist so zu verstehen, dass Festigkeitswerte einer frei wählbaren Anzahl von Kettfäden angezeigt werden können. So kann man beispielsweise die Gesamtzahl von angenommenen 5000 Kettfäden einer Fadenschicht in Gruppen zu je 500 Fäden unterteilen und die erwähnten Festigkeitswerte gruppenweise zur Anzeige bringen.

Fig. 3 zeigt einen schematischen Ausschnitt einer mit einem Dehnungsprüfer ausgerüsteten Webkettenknüpfmaschine, wobei nur die für das Verständnis der Erfindung erforderlichen Maschinenteile dargestellt sind. Hier erfolgt also keine Festigkeitsprüfung sondern eine Dehnungsprüfung, was eine wesentliche Vereinfachung bedeutet. Die frei gespannten Kettfäden Ka sind kurz vor zwei beabstandeten platten- oder schienenartigen Führungen 26 positioniert und werden zum Abteilen von ihrer Fadenschicht durch eine Abteilnadel 27 so weit in Richtung des Pfeiles A aus der Ebene der Fadenschicht ausgelenkt, dass sie in das Niveau des Mauls eines mit einem Haken versehenen Fadenzubringers 28 gelangen.

Dann wird der Fadenzubringer 28 in Richtung des Pfeiles B vom Rand der Fadenschicht wegbewegt, erfasst dabei mit seinem Haken den abgeteilten Faden Ka und zieht diesen von der Fadenschicht weg bis in die strichpunktiert eingezeichnete Lage. Bei dieser Verschiebung gelangt der Faden über die Führungen 26 auf einen zwischen den Führungen angeordneten Kraftmessteil 29, der im Bereich seiner oberen, den Faden führenden Kante mit einem druckempfindlichen Organ 30 ausgerüstet ist. Dieses Organ 30 kann beispielsweise durch eine Blattfeder oder durch einen am Kraftmessteil 29 federnd gelagerten, starren Druckaufnehmer gebildet sein.

Es wird in diesem Zusammenhang auf das schon erwähnte US-Patent 4,805,276 verwiesen, in dem offenbart ist, dass die das Organ 30 bildende Blattfeder oder, bei starr ausgebildetem Organ 30, die dieses tragende Feder mit Dehnungsmessstreifen oder mit auf Biegung empfindlichen piezoelektrischen Sensoren ausgerüstet ist.

Sobald der Faden den Teil 30 entlanggleitet, wird er unter zwei fingerartige Fadenspanner 31 geschoben, die beideitig des Teils 30 und parallel zu diesem angeordnet sind, und in ihrer Ruhestellung oberhalb des Fadens liegen. Wenn der Faden seine Endstellung auf dem Teil 30 erreicht hat, wird er vom Fadenzubringer 28 freigegeben, der anschliessend wieder in seine in der Figur dargestellte Ausgangsstellung bewegt wird.

Unmittelbar nach der Freigabe des Fadens werden die Fadenspanner 31, die auf um eine gemeinsame Welle 32 in Richtung der Pfeile C verschwenkbaren Armen 33 befestigt sind, nach unten verschwenkt und drücken den Faden nach unten in die gestrichelt eingezeichnete Stellung. Durch das Spannen des Fadens wird auf den Teil 30 eine Kraft ausgeübt, die ein entsprechendes Signal der genannten Sensoren erzeugt, welches ein Mass für die Dehnung des Fadens darstellt. Die Signale der Sensoren werden entsprechend der anhand von Fig. 2 beschriebenen Signalauswertung verarbeitet, wobei jedoch der Inkrementalgeber 23 nicht mehr unbedingt erforderlich ist.

## Patentansprüche

1. Verfahren zur Bestimmung von Festigkeitseigenschaften der Kettfäden einer Webkette, dadurch gekennzeichnet, dass die Kettfäden (Ka) als Fadenschicht aufgespannt, von der Fadenschicht einzeln abgeteilt und unter gleichzeitiger Messung von Zugkraft und/oder Dehnung gedehnt werden, und dass diese Operationen vor dem webprozess in einer Stufe des Webereivorbereitungsprozesses erfolgen, in der die Kettfäden in Form einer vorbereiteten Kettfadenschicht vorliegen.

2. Verfahren nach Anspruch 1, dadurch gekennzeichnet, dass die Bestimmung der Festigkeitseigenschaften beim Anknüpfen der Kettfäden (Ka) an eine auslaufende Webkette oder beim Einziehen der Kettfäden erfolgt und an dem nach dem Anknüpfen beziehungsweise Einziehen verbleibenden abgeschnittenen Fadenrest jedes Kettfadens vorgenommen wird.

3. Verfahren nach Anspruch 2, dadurch gekennzeichnet, dass für jeden Kettfaden (Ka) die Reisskraft und die Dehnung für verschiedene Punkte der Kraft-Dehnungslinie ermittelt wird.

4. Verfahren nach Anspruch 3, dadurch gekennzeichnet, dass aus den ermittelten Kraft- und Dehnungswerten weitere Grössen wie mittlere Reisskraft oder Dehnung und deren Schwankungen um einen Mittelwert abgeleitet und ausgegeben werden.

5. Verfahren nach Anspruch 4, dadurch gekennzeichnet, dass die genannten Werte und Grössen wahlweise einzeln oder für wählbare Gruppen von Kettfäden ausgegeben werden.

6. Vorrichtung zur Durchführung des Verfahrens nach Anspruch 1, gekennzeichnet durch Mittel (1, 2) zum Aufspannen der Kettfadenschicht, Mittel (4, 27) zum Abteilen der Kettfäden (Ka) und Mittel (FP, 30) zur Messung von Zugkraft und/oder Dehnung der abgeteilten Kettfäden, wobei die genannten Mittel zum Aufspannen der Kettfadenschicht Bestandteil einer Webkettenvorbereitungsmaschine, vorzugsweise einer Knüpfanlage oder einer Einziehanlage bilden.

7. Vorrichtung nach Anspruch 6, dadurch gekennzeichnet, dass die Mittel zum Aufspannen der Kettfadenschicht durch das Knüpfgestell (1, 2) einer Knüpfanlage gebildet sind.

8. Vorrichtung nach Anspruch 7, dadurch gekennzeichnet, dass die Knüpfmaschine der Knüpfanlage voneinander beabstandete Führungen (26) für die abgeteilten Fäden, Fadenzubringer (28) zum Transport dieser Fäden entlang den Führungen zu einem Kraftmessteil (29) und verstellbare Fadenspanner (31) zum Dehnen der Fäden aufweist.

9. Vorrichtung nach Anspruch 8, dadurch gekennzeichnet, dass der Kraftmessteil (29) platten- oder schienenförmig ausgebildet ist und eine mit einem druckempfindlichen Organ (30) versehene Führungskante für die Fäden aufweist, und dass die Fadenspanner (31) durch beidseitig des Kraftmessteils angeordnete und gegen dessen Fadenführungskante verschwenkbare, fingerartige Organe gebildet sind.

10. Vorrichtung nach Anspruch 7, dadurch gekennzeichnet, dass die Knüpfmaschine (KM) der Knüpfanlage mit einem Festigkeitsprüfer (FP) zur Bestimmung der Reissfestigkeit der Fäden und mit Mitteln (13, 14) zur Uebergabe der Fäden an den Festigkeitsprüfer versehen ist.

11. Vorrichtung nach Anspruch 10, dadurch gekennzeichnet, dass der Festigkeitsprüfer (FP) entlang einer Seitenfläche der Knüpfmaschine (KM) angeordnet ist, und zwar an derjenigen, welche zu der von dem die Fäden (Ka) verknüpfenden Organ (8) weiter beabstandeten Klemmschiene (2) benachbart ist.

12. Vorrichtung nach Anspruch 11, dadurch gekennzeichnet, dass der Festigkeitsprüfer (FP) zwei beabstandete und gegenläufig rotierbare Walzenpaare (W, W') sowie einen zwischen diesen angeordneten Kraftsensor (17) aufweist, dass jeder Faden im Festigkeitsprüfer zwischen den Walzenpaaren und über den Kraftsensor gespannt ist, und dass die Walzen der Walzenpaare so ausgebildet sind, dass zwischen ihnen abwechselnd ein offener und ein geschlossener Klemmspalt für einen Faden gebildet ist.

13. Vorrichtung nach Anspruch 12, dadurch gekennzeichnet, dass die Mittel zur Uebergabe der Fäden an den Festigkeitsprüfer (FP) durch eine entlang der vom Faden im Festigkeitsprüfer aufgespannten Bahn bewegbare Greifernadel (13) gebildet sind.

14. Vorrichtung nach Anspruch 13, dadurch gekennzeichnet, dass die Greifernadel (13) von einem flexiblen Antriebsband (14) getragen und an ihrer Spitze mit je einem Haken zur Mitnahme des von jeder Fadenschicht abgeteilten Fadens (Ka) versehen ist.

15. Vorrichtung nach Anspruch 14, gekennzeichnet durch einen Fadenauslenker (12) zum Auslenken des abgeteilten Fadens aus der Ebene seiner Fadenschicht in das Niveau des entsprechenden Hakens der Greifernadel (13).

16. Vorrichtung nach einem der Ansprüche 13 bis 15, gekennzeichnet durch Mittel (18) zum Spannen des von der Greifernadel (13) in den Festigkeitsprüfer (FP) eingezogenen Fadens.

17. Vorrichtung nach Anspruch 16, dadurch gekennzeichnet, dass die genannten Mittel durch eine in Einzugsrichtung des Fadens nach dem hinteren Walzenpaar (W') angeordnete Saugdüse (18) gebildet sind.

18. Vorrichtung nach einem der Ansprüche 13 bis 15, gekennzeichnet durch im Bereich des in Einzugsrichtung des Fadens vorderen Walzenpaars (W) angeordnete Mittel (19) zum Entfernen des nach der Festigkeitsprüfung im Festigkeitsprüfer (FP) verbleibenden Fadenrests.

19. Vorrichtung nach Anspruch 18, dadurch gekennzeichnet, dass die genannten Mittel durch eine rotierbare Ausziehscheibe (19) oder durch eine Saugdüse zur Mitnahme des Fadenrests nach dessen Freigabe durch das vordere Walzenpaar (W) gebildet sind.

## Claims

1. Method for determining strength properties of the warp threads of a warp, characterised in that the warp threads (Ka) are clamped as a thread layer, are divided off individually from the thread layer and are stretched with a simultaneous measurement of the tensile force and/or stretching, and in that these operations take place before the weaving process in a step of the weaving preparation process in which the warp threads are in the form of a prepared warp-thread layer.

2. Method according to Claim 1, characterised in that the determination of the strength properties takes place during the knotting of the warp threads (Ka) to a running-off warp or during the drawing in of the warp threads and is carried out on the cut-off thread residue of each warp thread which remains after the knotting or the drawing in.

3. Method according to Claim 2, characterised in that, for each warp thread (Ka), the tearing force and the stretching are determined for different points on the force/stretch line.

4. Method according to Claim 3, characterised in that further quantities, such as the average tearing force or stretching, and their fluctuations around an average value are derived from the force and stretch values determined and are outputted.

5. Method according to Claim 4, characterised in that said values and quantities are outputted either individually or for selectable groups of warp threads.

6. Device for carrying out the method according to Claim 1, characterised by means (1, 2) for clamping the warp-thread layer, means (4, 27) for dividing off the warp threads (Ka) and means (FP, 30) for measuring the tensile force and/or stretching of the divided-off warp threads, said means for clamping the warp-thread layer forming an integral part of a warp preparation machine, preferably of a knotting unit or of a drawing-in unit.

7. Device according to Claim 6, characterised in that the means for clamping the warp-thread layer are formed by the knotting stand (1, 2) of a knotting unit.

8. Device according to Claim 7, characterised in that the knotting machine of the knotting unit has guides (26), located at a distance from one another, for the divided-off threads, thread feeders (28) for transporting these threads along the guides to a force-measuring part (29) and adjustable thread tensioners (31) for stretching the threads.

9. Device according to Claim 8, characterised in that the force-measuring part (29) is made plate-shaped or rail-shaped and has a guide edge for the threads which is equipped with a pressure-sensitive member (30), and in that the thread tensioners (31) are formed by finger-like members which are arranged on both sides of the force-measuring part and which are pivotable against the thread-guide edge of the latter.

10. Device according to Claim 7, characterised in that the knotting machine (KM) of the knotting unit is equipped with a strength tester (FP) for determining the tearing strength of the threads and with means (13, 14) for transferring the threads to the strength tester.

11. Device according to Claim 10, characterised in that the strength tester (FP) is arranged along one side face of the knotting machine (KM), specifically on that which is adjacent to the clamping rail (2) located at a greater distance from the member (8) knotting the threads (Ka).

12. Device according to Claim 11, characterised in that the strength tester (FP) has two pairs of rollers (W, W') located at a distance from one another and rotatable in opposite directions and a force sensor (17) arranged between these, in that each thread in the strength tester is clamped between the pairs of rollers and via the force sensor, and in that the rollers in the pairs of rollers are designed so that an open nip and a closed nip for a thread is alternately formed between them.

13. Device according to Claim 12, characterised in that the means for transferring the threads to the strength tester (FP) are formed by a gripper needle (13) movable along the path covered by the thread in the strength tester.

14. Device according to Claim 13, characterised in that the gripper needle (13) is carried by a flexible driving band (14) and is equipped at its tip with a hook for taking up the thread (Ka) divided off from each thread layer.

15. Device according to Claim 14, characterised by a thread deflector (12) for deflecting the divided-off thread out of the plane of its thread layer to the level of the corresponding hook of the gripper needle (13).

16. Device according to one of Claims 13 to 15, characterised by means (18) for clamping the thread drawn into the strength tester (FP) by the gripper needle (13).

17. Device according to Claim 16, characterised in that said means are formed by a suction nozzle (18) arranged after the rear pair of rollers (W') in the drawing-in direction of the thread.

18. Device according to one of Claims 13 to 15, characterised by means (19), arranged in the region of the pair of rollers (W) at the front in the drawing-in direction of the thread, for removing the thread residue remaining in the strength tester (FP) after the strength test.

19. Device according to Claim 18, characterised in that said means are formed by a rotatable extractor disc (19) or by a suction nozzle for taking up the thread residue after its release by the front pair of rollers (W).

## Revendications

1. Procédé pour déterminer les caractéristiques de la résistance mécanique des fils de chaîne d'une chaîne de tissage, caractérisé en ce que les fils de chaînes (Ka) sont tendus sous forme d'une couche de fils, individuellement séparés de la couche de fils et étirés en mesurant simultanément la force de traction et/ou d'étirage, et en ce que ces opérations sont exécutées avant le processus de tissage à une phase, à laquelle les fils de chaîne se présentent sous forme d'une couche de fils de chaîne préparée.

2. Procédé selon la revendication 1, caractérisé en ce que la détermination des caractéristiques de résistance mécanique se fait lors du rattachement des fils de chaîne (Ka) à une chaîne de tissage qui se termine ou lors de la rentrée des fils de chaîne et est effectuée pour le restant de fil découpé de chaque fil de chaîne, qui reste après le rattachement ou la rentrée.

3. Procédé selon la revendication 2, caractérisé en ce que pour chaque fil de chaîne (Ka) est déterminée la résistance à la rupture et l'étirage pour différents points de la courbe caractéristique force-étirage.

4. Procédé selon la revendication 3, caractérisé en ce que d'autres grandeurs sont déterminées à partir des valeurs de force et d'étirage, qui ont été déterminées, telles que la résistance moyenne à la rupture ou l'étirage moyen et leurs variations autour d'une valeur moyenne sont dérivées et indiquées.

5. Procédé selon la revendication 4, caractérisé en ce que les valeurs et grandeurs précitées sont fournies, au choix, individuellement ou pour des groupes qui peuvent être choisis, de fils de chaîne.

6. Dispositif pour la mise en oeuvre du procédé selon la revendication 1, caractérisé par des moyens (1, 2) pour la formation de la couche de fils de chaîne, des moyens (4, 27) pour la séparation de fils de chaîne (Ka) et des moyens (FP, 30) pour la mesure de la force de traction et/ou de l'étirage des fils de chaîne séparés, lesdits moyens pour la formation de la couche de fils de chaîne faisant partie d'une machine de préparation de chaînes de disque, de préférence d'une installation à nouer ou d'une installation d'alimentation.

7. Dispositif selon la revendication 6, caractérisé en ce que les moyens pour la formation de la couche de fils de chaîne sont formés par un châssis noueur (1, 2) d'une installation à nouer.

8. Dispositif selon la revendication 7, caractérisé en ce que la machine à nouer de l'installation à nouer comprend des guides (26) présentant un écart les uns des autres pour les fils séparés, des fournisseurs de fils (28) pour le transport de ces fils le long des guides à une pièce de mesure de force (29) et des dispositifs tendeurs de fil (31) réglable pour étirer les fils.

9. Dispositif selon la revendication 8, caractérisé en ce que la pièce de mesure de force (29) est réalisée sous forme d'une plaque ou d'un rail et comprend une arête de guidage pour les fils, pourvue d'un organe (30) sensible à la pression, et en ce que les tendeurs de fils (31) sont formés par des organes en forme de doigts, disposés aux deux côtés de la pièce de mesure de force et susceptibles d'un pivotement vers l'arête de guidage de fil.

10. Dispositif selon la revendication 7, caractérisé en ce que la machine à nouer (KM) d'une installation à nouer est pourvue d'un dispositif de contrôle de la résistance mécanique (FP) pour déterminer la résistance à la rupture des fils et de moyens (13, 14) pour le transfert des fils au dispositif de contrôle de résistance.

11. Dispositif selon la revendication 10, caractérisé en ce que le contrôleur de résistance (FP) est disposé le long d'une face latérale de la machine à nouer (KM), à savoir à celle qui est voisine du rail de serrage (2) plus éloigné de l'organe (8) nouant les fils (Ka).

12. Dispositif selon la revendication 11, caractérisé en ce que le contrôleur de résistance (FP) comprend deux paires de cylindres (W, W') présentant un certain écart l'un de l'autre et susceptibles de tourner dans des sens inverses, ainsi qu'un capteur de force (17) disposé entre ceux-ci, que chaque fil dans le contrôleur de résistance est tendu entre les paires de cylindres et sur le capteur de force, et que les cylindres des paires de cylindres sont configurés de telle façon qu'entre eux soit formée une fente de serrage pour le fil, qui est alternativement ouverte ou fermée.

13. Dispositif selon la revendication 12, caractérisé en ce que les moyens pour le transfert de fils au contrôleur de résistance (FP) sont formés par une aiguille de préhension (13) déplaçable le long de la trajectoire établie par le fil dans le contrôleur de résistance.

14. Dispositif selon la revendication 13, caractérisé en ce que l'aiguille de préhension (13) est portée par une bande d'entraînement flexible (14) et est pourvue à sa pointe d'un crochet pour l'entraînement du fil (Ka) séparé de chaque couche de fils.

15. Dispositif selon la revendication 14, caractérisé par un organe d'excursion de fil (12) pour l'excursion du fil séparé du plan de sa couche de fils au niveau du crochet correspondant de l'aiguille de préhension (13).

16. Dispositif selon l'une des revendications 13 à 15, caractérisé par des moyens (18) pour tendre le fil rentré par l'aiguille de préhension (13) dans le contrôleur de résistance (FP).

17. Dispositif selon la revendication 16, caractérisé en ce que les moyens précités sont formés par une tuyère d'aspiration (18) disposée dans la direction de rentrée du fil après la paire de cylindres arrière (W').

18. Dispositif selon l'une des revendications 13 à 15, caractérisé par des moyens (19) disposés dans la zone de la paire de cylindres avant (W) dans la direction de rentrée des fils, pour l'évacuation du restant de fil demeurant dans le contrôleur de résistance (FP) après le contrôle de résistance.

19. Dispositif selon la revendication 18, caractérisé en ce que les moyens précités sont formés par un disque d'extraction tournant (19) ou par une tuyère d'aspiration pour l'entraînement du restant de fil après sa libération par la paire de cylindres avant (W).
